# FASCICULE DE BREVET EUROPEEN

(11) **EP 0 532 430 B1**
(45) Date de publication et mention de la délivrance du brevet: **28.02.1996**
(21) Numéro de dépôt: 92420262.5
(22) Date de dépôt: 31.07.1992
(51) Int. Cl.: G01N 33/569, G01N 33/577

(54) **Réactif d'identification des bactéries de l'espèce staphyloccocus aureus**
Reagenz zur Identifizierung Bakterien der Spezies Staphylococcus aureus
Reagent to identify bacteria of the species Staphylococcus aureus

(30) Priorité: 02.08.1991 FR 9110105
(43) Date de publication de la demande: 17.03.1993
(73) Titulaire: BIO MERIEUX, Société anonyme, F-69280 Marcy l'Etoile (FR)
(72) Inventeur: Merlin, Sylviane, F-69300 Caluire (FR); Piga, Nadia, F-69008 Lyon (FR)
(74) Mandataire: Guerre, Dominique

(56) Documents cités:
- WO-A-88/02028
- PATHOLOGIE BIOLOGIE vol. 27, no. 5, 1979, PARIS FRANCE pages 297 - 299 J.P. FLANDROIS ET AL. 'Synthèse de la protéine à de surface et de l àntigène de type 18 chez Staphylococcus aureus en présence de méthicilline.'
- NARURE vol. 213, 18 Mars 1987, LONDON UK page 1137 Y.A. CHABBERT ET AL. 'Correlation between methillicin resistance and serotype in Staphylococcus.'

## Description

La présente invention concerne la détection, l'identification, et le dénombrement des bactéries de l'espèce *Staphyloccocus aureus*.

On connaît déjà des réactifs d'identification de l'espèce *Staphyloccocus aureus*, comprenant un composé réactif vis-à-vis d'un antigène desdites bactéries, à savoir la protéine A du Staphyloccocus, et/ou un autre composé à base de fibrinogène susceptible de réagir avec le facteur d'affinité pour le fibrinogène desdites bactéries.

Dans le premier cas, le réactif d'identification est une immuno-globuline dont le fragment Fc se fixe, par un procédé non immunologique, sur la protéine A du Staphyloccoque, en laissant libre la partie Fab de la même immuno-globuline.

La protéine A est un antigène de nature protéique, constituant externe de la paroi de la majorité des souches de *Staphyloccocus aureus* d'origine humaine (environ 90 %). Lorsqu'on met en présence des souches de *Staphyloccocus aureus* possédant la protéine A et des hématies de mouton, sensibilisées avec du sérum de lapin anti-hématies de mouton ou des particules de latex sur lesquelles sont fixées des gammaglobulines, on observe en quelques minutes une agglutination visible à l'oeil nu.

Dans le second cas, le réactif d'identification est à base de fibrinogène, c'est-à-dire est ou contient du fibrinogène réactif, ou à base d'un produit dérivé, c'est-à-dire conservant une réactivité comparable au fibrinogène, mais modifié chimiquement.

Le facteur d'affinité pour le fibrinogène, qui est fixé à la surface du *Staphyloccocus aureus*, réagit directement avec le fibrinogène. Ce facteur d'affinité pour le fibrinogène peut être mis en évidence en mettant en contact la souche à étudier et des hématies de mouton sensibilisées par du fibrinogène, ou des particules de latex sur lesquelles a été fixé du fibrinogène. Ainsi, si la souche à étudier possède le facteur d'affinité pour le fibrinogène, on observe une agglutination visible à l'oeil nu. On peut citer à titre d'exemple, le réactif commercialisé par la Société BIO MERIEUX sous la dénomination "Staphyslide", qui utilise pour la détection de *Staphyloccocus aureus* la recherche du facteur d'affinité pour le fibrinogène, par mise en contact de la souche à étudier avec des hématies sensibilisées par le fibrinogène, et l'observation d'une hémagglutination passive.

Cependant, différentes études ont montré qu'un certain nombre de souches de *Staphyloccocus aureus*, et particulièrement les souches résistantes à la méthicilline, ne sont pas identifiées par ces réactifs.

Les souches de Staphyloccocus aureus résistantes à la méthicilline ont des caractères qui les opposent aux souches de la même espèce, sensibles à l'antibiotique. On a notamment observé des anomalies dans la production de la protéine A, dont la synthèse peut être diminuée en quantité, voire parfois totalement absente (cf FLANDROIS J.P., FLEURETTE J., EYRAUD F., Détection de la protéine A de *Staphyloccocus aureus* par hémagglutination conditionnée, Ann. Biol. clin., 1975, 33, 365-368 et LIND I., Acta path. microbiol. scand., 1972, 80, section B, 702-708), ou bien la protéine A peut être libérée dans le milieu de culture et non plus fixée à la paroi (cf LINDMARK R., MOVITZ J., SJOQUIST J., Eur. J. Biochem, 1977, 74, 623-628). En conséquence, les réactifs du commerce ne permettent pas de détecter les souches résistantes à la méthicilline, soit parce que la protéine A est absente de la paroi de ces souches, soit parce qu'elle est produite en quantité trop faible pour qu'il y ait entre cette protéine et des particules de latex ou des hématies sensibilisées avec une gammaglobuline, une réaction suffisante pour provoquer une agglutination bactérienne.

Par ailleurs, d'autres études ont montré que la majorité des souches de *Staphyloccocus aureus* résistantes à la méthicilline ne possèdent pas le facteur d'affinité pour le fibrinogène, en raison d'anomalies de la paroi bactérienne (cf R. LALLY et B. WOLLFREY, Eur. J. Clin. Microbiol., Vol.3, N° 2, pages 151-152 (1984)). En conséquence, les souches ne possédant pas le facteur d'affinité pour le fibrinogène ne peuvent être détectées par les réactifs conventionnels.

Les travaux de Y.A. CHABBERT et J. PILLET (Nature (LOND.), 1967, 213, 1137) ont montré que les souches bactériennes de *Staphyloccocus aureus* résistantes à la méthicilline possèdent un antigène de paroi spécifique, appelé antigène 18.

La présente invention a pour objet un réactif susceptible de réagir avec au moins l'un quelconque des déterminants suivants, à savoir le facteur d'affinité pour le fibrinogène, la protéine A, et l'antigène 18, et partant susceptible de détecter les souches bactériennes de *Staphyloccocus aureus* quels que soient leurs sérotypes.

Selon la présente invention, on a tout d'abord découvert que l'antigène 18 est une glycoprotéine d'un poids moléculaire compris entre 12500 et 13000 daltons, et ayant un point isoélectrique compris entre 4,85 et 5,2.

A partir de cette caractérisation de l'antigène 18, on a ensuite trouvé qu'un tel réactif pouvait être obtenu, en choisissant et sélectionnant par des moyens traditionnels et usuels de l'immunologie, un composé réactif du type immuno-globuline, comprenant à la fois un fragment Fab spécifiquement immuno-réactif vis-à-vis de l'antigène 18 des souches méthicilline-résistantes, et un fragment Fc susceptible comme précédemment de se fixer sur la protéine A des bactéries *Staphyloccocus aureus* en général.

Par "composé", on entend un matériel du type immuno-globuline, fixé ou non sur un support, et comprenant de toutes façons deux fragments Fab et Fc, accessibles aux bactéries à identifier ou détecter.

Préférentiellement, le composé immunologiquement réactif est ou comprend un anticorps comportant les fragments Fab et Fc, fixé ou non sur un support ou substrat.

Les anticorps utilisés selon la présente invention peuvent être des anticorps animaux ou humains, polyclonaux ou monoclonaux, suceptibles d'être reconnus par affinité par la protéine A, et de former un complexe antigène-anticorps avec l'antigène 18. Ces anticorps sont notamment des anticorps de classe IgG.

Dans le cas d'anticorps polyclonaux, on peut utiliser pour la préparation des réactifs selon l'invention, un plasma humain ou animal immunisé, contenant ces anticorps, ou des anticorps purifiés selon les techniques classiques.

De préférence, les anticorps sont des anticorps monoclonaux animaux, tels que des anticorps monoclonaux de souris, et on peut utiliser comme réactif un surnageant de culture d'hybridome ou du liquide d'ascite préparé sur souris, ou des anticorps monoclonaux à l'état purifié.

Préférentiellement, un anticorps monoclonal selon l'invention est obtenu à partir de la lignée cellulaire hybridome déposée le 26 juillet 1991, en Grande-Bretagne, sous le N° 91072636 auprès de l'ECACC (European Collection of Animal Cell Cultures), sous les dispositions du Traité de Budapest.

Cette lignée cellulaire est revendiquée en tant que telle, ainsi que toute lignée cellulaire dérivée, c'est-à-dire susceptible de produire des anticorps présentant les mêmes caractéristiques immuno-chimiques que celles décrites selon la présente invention ; toute progéniture de l'hybridome identifié précédemment fait partie de la présente invention.

Qu'il s'agisse des anticorps polyclonaux ou monoclonaux selon l'invention, la protection revendiquée s'étend aux anticorps présentant une réaction croisée avec ceux-ci.

Préférentiellement, l'immuno-réactif selon l'invention comprend aussi un composé à base de fibrinogène, fixé ou non sur un support ou substrat, à savoir éventuellement le même support que celui de l'anticorps. Le fibrinogène peut être un plasma humain ou animal, normal ou hyperimmunisé, ou du fibrinogène purifié selon les techniques classiques.

Toutes sortes de support ou substrat peuvent être envisagées selon l'invention.

On peut ainsi utiliser des particules en suspension. Ces particules sont notamment des particules de latex, telles que des billes de polystyrène ou des particules similaires, ayant de préférence une taille inférieure à 2 µm. A titre d'exemple, on peut citer les particules Estapor, commercialisées par la Société RHONE-POULENC, telles que :
- des particules de polystyrène K080, ayant un diamètre de 0,8 µm
- des particules de polystyrène K109, ayant un diamètre de 0,8 µm
- des particules de polystyrène ayant des groupes carboxyliques, PSI 480, ayant un diamètre de 0,8 µm.

On peut également utiliser des gels magnétiques, tels que les gels de polyacrylamide et/ou d'agarose contenant des particules magnétiques. On peut en outre utiliser des gels tels que Ultrogel et Magnogel (marques de commerce) de la Société IBF.

Le support ou substrat, utilisé dans un réactif selon la présente invention, peut être également des hématies de mouton, sensibilisées avec du fibrinogène.

Le support ou substrat peut être sous la forme d'une plaque, d'un cône, d'une barrette, par exemple de polystyrène, ou d'un copolymère à base de styrène, d'un tube de verre ou analogue.

La fixation d'anticorps et de fibrinogène sur des particules en suspension, notamment de latex, peut être réalisée selon l'une des techniques suivantes :
- par adsorption passive, la fixation pouvant être spontanée, au cours d'une incubation des particules de latex dans une solution contenant les anticorps et le fibrinogène ; une incubation par exemple d'environ 2 heures à 20° C est souvent suffisante ;
- par covalence, la fixation pouvant être réalisée en créant une liaison covalente entre les anticorps et les groupements carboxyliques présents sur certaines particules du latex ; il est possible par exemple d'utiliser un carbodiimide pour créer la liaison covalente.

Lorsque le support est constitué d'hématies, celles-ci sont au préalable sensibilisées par du fibrinogène selon des techniques classiques.

La concentration des anticorps à fixer sur les particules de latex, qui doit être déterminée pour chaque anticorps selon les méthodes connues, est habituellement comprise entre 2 µg et 20 µg de protéines anticorps par millilitre de latex en solution. De préférence, la concentration est d'environ 5 µg/ml de latex en solution.

Dans un réactif selon l'invention, l'anticorps et le fibrinogène peuvent être fixés sur une suspension unique de particules, par exemple de latex, ou bien être fixés sur des suspensions de particules respectivement différentes, telles que des hématies et des particules de latex, ou différents latex qui sont ensuite mélangés pour constituer le réactif.

### Exemple 1 : caractérisation de l'antigène 18

L'antigène 18 a été caractérisé selon les techniques habituelles, à partir de la souche bactérienne de Staphylococcus aureus J. PILLET 64.002, déposée à la collection de l'Institut Pasteur sous le numéro 65.25. On a réalisé une autolyse d'une pâte bactérienne de la souche P 18. Le culot final a été repris dans l'urée 8M, dialysé contre H₂O à pH = 7, congelé et lyophilisé. On a a effectué un pool de 5 électrofocalisations en colonnes dans de l'urée 6M, puis une étape de dessalage sur une colonne chromatographique GF 05 (non commercial). Le produit obtenu est filtré sur un filtre Millex ( marque de commerce) 0,22 µ, puis lyophilisé. Le produit lyophilisé a été repris dans un tampon, puis soumis à une chromatographie d'affinité sépharose/protéine A, et le pool obtenu a été soumis à une étape de dessalage sur colonne chromatographique GF 05, puis lyophilisé.

Le produit obtenu présente les caractéristiques suivantes : c'est une glycoprotéine, d'un poids moléculaire compris entre 12500 et 13000 daltons, ayant un point isoélectrique compris entre 4,85 et 5,2.

A faible concentration, le produit obtenu inhibe totalement la réaction entre le sérum préparé selon la méthode décrite par J. PILLET et al, dans la publication de 1967 précédemment citée, et la souche déposée à la collection de l'Institut Pasteur sous le N° 65.25.

Bien entendu, il est dans les connaissances de l'homme de métier d'utiliser une autre méthode de purification, pouvant conduire à une protéine possédant les mêmes propriétés immunologiques, mais des propriétés physico-chimiques différentes.

### Exemple 2 : obtention des anticorps monoclonaux

Des anticorps monoclonaux anti-glycoprotéine 18 ont été préparés selon une technique adaptée de Galfre et al (Galfre G. et al. (1977) Nature, 266 : 522-550) en utilisant comme lignée cellulaire initiale pour la fusion la lignée du myélome Sp2/OAg 14 (ATCC CRL 1581). La fusion est effectuée avec les cellules de rate de souris, de l'espèce BALB/c et de la souche BALB/cByJ1co, immunisées par une souche de *Staphylococcus aureus* sérotype 18 selon les techniques classiques. Les clones obtenus ont été criblés par techniques immunoenzymatiques (ELISA). Six clones ont été sélectionnés, qui ont été réinjectés par voie intrapéritonéale à des souris préparées au préalable pour la production de liquide d'ascite. Chaque anticorps monoclonal a été utilisé pour sensibiliser des particules de latex, et a été testé pour sa spécificité à reconnaître la glycoprotéine 18 de *Staphylococcus aureus.* L'anticorps monoclonal dénommé P25A6B9A9 par le Déposant a été choisi en raison de sa spécificité, cet anticorps a reçu le numéro d'accès 91072636, attribué par l'ECACC.

La lignée cellulaire hybridome conduisant à l'anticorps monoclonal P25A6B9A9 a fait l'objet du dépôt auprès de l'ECACC, précédemment identifié.

### Exemple 3 : préparation d'hématies sensibilisées avec du fibrinogène.

a) Une suspension d'hématies de mouton a été amenée à la concentration de 8 % dans un tampon NaCl (0,137 M). Un volume de cette suspension a été mélangé avec un volume égal d'aldéhyde pyruvique amené à une concentration de 3 % dans un tampon citrate de sodium (50 g/l), puis mis à incuber pendant 18 heures à température ambiante. Le mélange a ensuite été centrifugé à 3.000 tours/min, et on a éliminé le surnageant. Le culot a été repris avec NaCl (0,137 M). On a réalisé 3 lavages successifs avec NaCl (0,137 M) par centrifugation. Le culot final a été repris avec du citrate de sodium à 5 % (3 à 4 volumes/volume du culot), et la suspension des hématies a été amenée à la concentration de 8 % dans le tampon citrate de sodium 5 %.
b) Un volume de la suspension d'hématies à 8 % dans le tampon citrate de sodium obtenue en (a) a été mélangé avec un volume égal d'aldéhyde formique amené à la concentration de 3 % dans le tampon citrate de sodium 5 %. Le mélange a été soumis à incubation pendant 5 jours à environ 20° C. Après incubation, le mélange a été centrifugé à 3.000 tours/min, son surnageant a été éliminé et le culot a été repris avec un tampon NaCl (0,137 M). Trois lavages successifs ont été ensuite effectués par centrifugation dans les conditions ci-dessus, et le culot final a été repris dans un tampon phosphate (0,15 M, pH = 7,2) et azide de sodium (1 g/l) (3 à 4 volumes de tampon/volume du culot). On a effectué une suspension des hématies à une concentration de 10 % dans le tampon phosphate (0,15 M, pH = 7,2) et azide de sodium (1 g/l).
c) La suspension d'hématies obtenue en (b) a été lavée par centrifugation à 3.000 tours/min dans un tampon phosphate NaCl (0,15 M, pH = 7,2). Le culot a été repris avec du tampon phosphate NaCl (0,15 M, pH = 7,2), Un volume d'hématies ainsi stabilisées a été mélangé à un volume de fibrinogène (0,9 mg/ml). Le mélange a été incubé à 37° C pendant 35 min avec agitation, pour effectuer la sensibilisation des hématies. Après incubation, le mélange a été centrifugé à 3.000 tours/min pour éliminer le surnageant, et le culot a été repris avec du tampon phosphate NaCl (0,15M, pH = 7,2) (6 à 8, volumes de tampon/volume de culot). On a effectué ensuite 3 lavages successifs par centrifugation dans les conditions ci-dessus, et le culot final a été repris avec un tampon phosphate NaCl (0,15 M, pH = 7,2), sérum albumine de bovin (1 g/l) et azide de sodium (1 g/l). On a effectué une suspension des hématies ainsi obtenues à une concentration de 10 % dans le tampon phosphate NaCl (0,15 M, pH = 7,2), sérum albumine de bovin (1 g/l) et azide de sodium (1 g/l).

### Exemple 4 : préparation d'un réactif témoin

On a effectué la préparation d'un réactif témoin selon le protocole décrit dans l'exemple 3, à l'exception de la sensibilisation qui est effectuée dans un tampon phosphate NaCl (0,15 M, pH = 7,2) sans fibrinogène.

### Exemple 5 : préparation de particules de latex sensibilisées par un anticorps monoclonal de souris anti-Staphylococcus aureus sérotype 18.

Un liquide d'ascite préparé sur souris selon le protocole décrit dans l'exemple 2 a été défibriné par dialyse contre du chlorure de calcium (25mM). Une suspension de latex (Estapore, K 0.80) à une concentration de 10 % a été sensibilisée avec l'ascite défibriné dans un tampon glycocolle (0,08 M) NaCl (0,12 M) (pH = 5,5), et en présence d'un détergent laurylsulfate (2,5 mg %). Le mélange est soumis à agitation pendant 2 heures à environ 20° C. Après sensibilisation, le mélange a été stabilisé pendant 30 min à 20° C, avec agitation par addition d'un volume égal de tampon glycocolle (0,08 M) NaCl (0,12 M) (pH = 7) contenant de la sérum-albumine de bovin (0,125 g %). La concentration finale du latex est de 0,5 %. Des conservateurs merthiolate de sodium (0,02 g %) et azide de sodium (0,1 g %) ont été ajoutés.

### Exemple 6 : préparation d'un latex témoin

On a effectué la préparation d'un latex témoin selon le processus décrit dans l'exemple 5, à l'exception de la sensibilisation qui a été effectuée dans un tampon glycocolle (0,08 M), NaCl (0,12 M) (pH = 5,5), sans liquide d'ascite.

### Exemple 7 : préparation du réactif final pour la détection de Staphylococcus aureus

Le réactif a été préparé selon le protocole décrit dans l'exemple 3, et mélangé avec le réactif préparé selon le protocole décrit dans l'exemple 5, dans les proportions suivantes : 1 volume d'hématies sensibilisées avec du fibrinogène, pour 2 volumes de latex sensibilisé par un anticorps monoclonal.

### Exemple 8 : préparation d'un réactif témoin

On effectue le mélange du réactif préparé selon le protocole décrit dans l'exemple 4 avec le réactif préparé selon le protocole décrit dans l'exemple 6 selon les proportions suivantes : un volume d'hématies non sensibilisées par la fibrinogène pour deux volumes de latex non sensibilisé par du liquide d'ascite.

### Exemple 9 :

Des essais ont été réalisés pour mettre en évidence la sensibilité et la spécificité du réactif de la présente invention.

### a) Sensibilité du réactif de l'invention

On a réalisé une étude pour comparer le pouvoir agglutinant d'un réactif selon la présente invention avec celui d'un réactif commercial (Staphyslide, commercialisé par la Société BIO MERIEUX).

On a effectué une comparaison entre le réactif du commerce et celui de la présente invention, à partir de 204 souches résistantes à la méthicilline. Sur les 204 souches, 137 sont identifiées par le réactif commercial, ce qui correspond à une sensibilité de 67,16 %, alors que le réactif de la présente invention permet d'identifier 186 souches, ce qui correspond à une sensibilité du réactif sur les souches *Staphylococcus aureus* résistantes à la méthicilline, de 91,17 %.

Parmi ces souches résistantes à la méthicilline, 67 ne possédaient pas le facteur d'affinité pour le fibrinogène, et ne pouvaient donc être identifiées par le réactif commercial. Ces 67 souches ont été testées avec le réactif de la présente invention, et on a obtenu une agglutination avec 49 d'entre elles. Ainsi, 73,13% des souches méthicilline-résistantes qui n'agglutinaient pas avec le réactif du commerce sont identifiées par le réactif de la présente invention.

Au total, la comparaison a été réalisée avec un total de 330 souches de *Staphylococcus aureus*, parmi lesquelles 204 souches étaient résistantes à la méthicilline et 126 sensibles à l'antibiotique. Alors que le réactif commercial n'agglutinait que 262 souches sur 330, le réactif préparé selon l'invention agglutine 312 souches sur les 330. L'étude portant sur 330 souches dont 72,7 % étaient résistantes à la méthicilline a montré que la sensibilité du réactif de l'invention est donc de 94,54 %, alors que celle du réactif du commerce n'était que de 79,39 %.

Dans le cadre d'une utilisation en diagnostic médical, la sensibilité du réactif est reliée à la fréquence des isolements des souches résistantes à la méthicilline ; si cette fréquence est de 10 %, alors la sensibilité du réactif sera environ de 99 %.

En outre, la rapidité et l'intensité de l'agglutination des souches qui étaient déjà agglutinées par le réactif commercial sont très nettement améliorées par l'utilisation du réactif préparé selon l'invention.

### b) Spécificité du réactif de la présente invention

On a réalisé une étude pour déterminer la spécificité du réactif de la présente invention, en établissant une comparaison avec le réactif commercialisé Staphyslide. Cette comparaison a été effectuée à partir de 89 souches de Staphylococcus non-aureus.

Les résultats sont présentés dans le tableau ci-dessous.

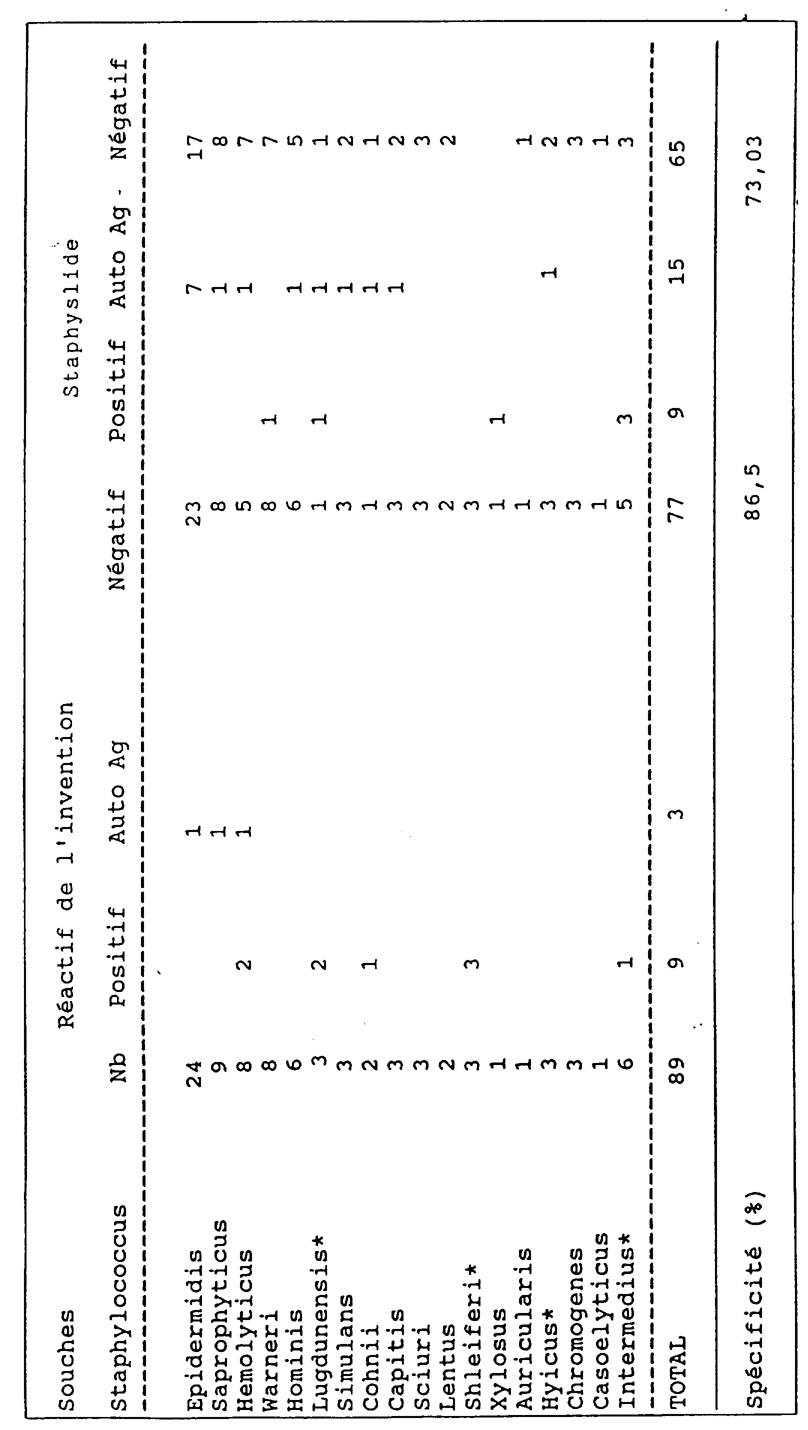

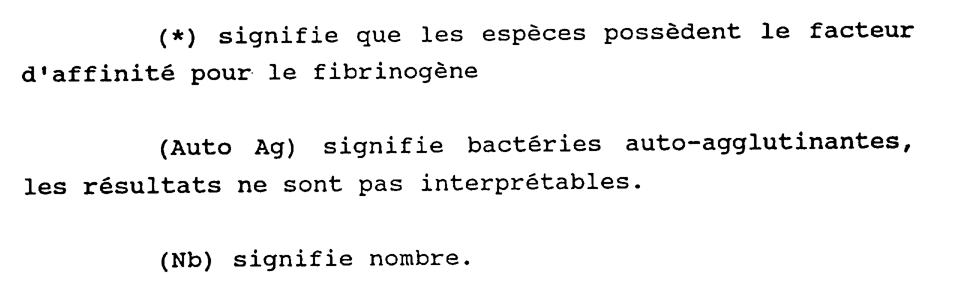

Comme cela ressort du tableau ci-dessus, le réactif de l'invention présente une meilleure spécificité que le réactif commercial.

Le réactif de la présente invention permet de reconnaître les souches de *Staphylococcus aureus* qui ne sont pas reconnues par les réactifs agglutinants du commerce, et parmi lesquelles prédominent les souches résistantes à la méthicilline.

L'intérêt du réactif de l'invention est donc majeur, compte tenu des problèmes que posent ces souches qui sont le plus souvent multi-résistantes.

## Revendications (Revendications pour l'(les) Etat(s) contractant(s) suivant(s): AT, BE, CH, DE, FR, GB, IT, LI)

1. Réactif d'identification des bactéries de l'espèce *Staphyloccocus aureus,* notamment des souches résistant à la méthicilline, du genre comprenant un composé réactif, de type immuno-globuline, comprenant un fragment Fc susceptible de se fixer sur la protéine A desdites bactéries, et éventuellement un autre composé à base de fibrinogène ou dérivé, susceptible de réagir avec le facteur d'affinité pour le fibrinogène desdites bactéries, **caractérisé en ce que** le composé réactif comprend, outre le fragment Fc, un fragment Fab spécifiquement immuno-réactif vis-à-vis de l'antigène 18 des souches méthicilline-résistantes.

2. Réactif selon la revendication 1, caractérisé en ce que le composé réactif comprend un anticorps comportant lesdits fragments Fab et Fc, éventuellement fixé à un support ou substrat.

3. Réactif selon la revendication 2, caratérisé en ce que l'anticorps est fixé à des particules d'un polymère, notamment un élastomère, par exemple un latex, ou à des particules magnétiques.

4. Réactif selon la revendication 2, caractérisé en ce que l'anticorps est fixé à des hématies humaines ou animales.

5. Réactif selon la revendication 1, caractérisé en ce qu'il comprend ledit autre composé à base de fibrinogène ou dérivé, lui-même fixé à un support ou substrat.

6. Réactif selon la revendication 1, comportant le composé réactif et l'autre composé à base de fibrinogène, fixés à un même support ou substrat, le composé réactif étant un anticorps.

7. Réactif selon la revendication 1, dans lequel le composé réactif est un anticorps, et l'autre composé à base de fibrinogène est présent, ces deux composés étant respectivement fixés à des supports différents, notamment des particules de latex et des hématies respectivement.

8. Anticorps du type immuno-globuline, spécifique aux bactéries de l'espèce *Staphylococcus aureus,* et notamment des souches résistant à la méthicilline, caractérisé en ce qu'il est du type immuno-globuline, notamment de la classe IgG, et comprend à la fois un fragment Fab spécifiquement immuno-réactif vis-à-vis de l'antigène 18 des souches méthicilline-résistantes, et un fragment Fc susceptible de se fixer sur la protéine A desdites bactéries.

9. Anticorps selon la revendication 8, du type polyclonal, ou tout autre anticorps présentant une réaction immunologique croisée avec ledit anticorps, caractérisé en ce qu'il est obtenu en immunisant un animal avec l'antigène 18, et en extrayant l'anticorps du plasma ainsi obtenu.

10. Anticorps selon la revendication 8, du type monoclonal, ou tout autre anticorps présentant une réaction immunologique croisée avec ledit anticorps, caractérisé en ce qu'il est obtenu à partir de la lignée cellulaire hybridome déposée le 26 juillet 1991, sous le N° 91072636 auprès de l'ECACC.

11. Lignée cellulaire hybridome, telle que déposée le 26 juillet 1991, sous le N° 91072636 auprès de l'ECACC, ou toute autre lignée cellulaire dérivée, ou toute progéniture de cet hybridome, pour autant que ces lignées et progéniture soient capables de produire un anticorps selon la revendication 10.

12. Lignée cellulaire hybridome résultant de la fusion d'une cellule provenant de lignée cellulaire Sp2/OAg14 (ATCC CRL 1581), avec une cellule de rate de souris provenant d'une souris immunisée avec le sérotype 18 de la souche de *Staphyloccocus aureus,* susceptible de produire un anticorps selon la revendication 10.

13. Procédé d'obtention d'un anticorps selon la revendication 10, caractérisé en ce qu'on injecte à une espèce animale dont est issu l'hybridome une culture des cellules telles que déposées le 26 juillet 1991, sous le N° 91072636 auprès de l'ECACC, et on extrait l'anticorps du liquide ascite récolté sur ladite espèce.

14. Procédé selon la revendication 13, du type agglutination.

15. Procédé d'identification des bactéries de l'espèce *Staphylococcus aureus,* y compris les souches résistant à la méthicilline, selon lequel on forme un complexe entre un composé réactif et un antigène desdites bactéries, caractérisé en ce que le composé réactif est du type immuno-globuline, et comprend à la fois un fragment Fab spécifiquement immuno-réactif vis-à-vis de l'antigène 18 des souches méthicilline-résistantes, et un fragment Fc susceptible de se fixer sur la protéine A desdites bactéries.

## Revendications (Revendications pour l'(les) Etat(s) contractant(s) suivant(s): ES)

1. Procédé d'identification des bactéries de l'espèce *Staphylococcus aureus,* y compris les souches résistant à la méthicilline, selon lequel on forme un complexe entre un composé réactif et un antigène desdites bactéries, et éventuellement on forme un complexe entre un autre composé à base de fibrinogène ou dérivé, susceptible de réagir avec le facteur d'affinité pour le fibrinogène desdites bactéries, caractérisé en ce que le composé réactif est du type immuno-globuline, et comprend à la fois un fragment Fab spécifiquement immuno-réactif vis-à-vis de l'antigène 18 des souches méthicilline-résistantes, et un fragment Fc susceptible de se fixer sur la protéine A desdites bactéries.

2. Procédé selon la revendication 1, caractérisé en ce que le composé réactif comprend un anticorps comportant lesdits fragments Fab et Fc, éventuellement fixé à un support ou substrat.

3. Procédé selon la revendication 2, caratérisé en ce que l'anticorps est fixé à des particules d'un polymère, notamment un élastomère, par exemple un latex, ou à des particules magnétiques.

4. Procédé selon la revendication 2, caractérisé en ce que l'anticorps est fixé à des hématies humaines ou animales.

5. Procédé selon la revendication 1, caractérisé en ce que ledit autre composé à base de fibrinogène ou dérivé est lui-même fixé à un support ou substrat.

6. Procédé selon la revendication 1, caractérisé en ce que le composé réactif et l'autre composé à base de fibrinogène sont fixés à un même support ou substrat, et en ce que le composé réactif est un anticorps.

7. Procédé selon la revendication 1, selon lequel le composé réactif est un anticorps, et l'autre composé à base de fibrinogène est présent, ces deux composés étant respectivement fixés à des supports différents, notamment des particules de latex et des hématies respectivement.

8. Procédé d'obtention d'un anticorps du type immuno-globuline, spécifique aux bactéries de l'espèce *Staphylococcus aureus,* et notamment des souches résistant à la méthicilline, caractérisé en ce qu'on immunise un animal avec l'antigène 18, et on extrait l'anticorps du plasma ainsi obtenu, et en ce que ledit anticorps est du type immuno-globuline, notamment de la classe IgG, et comprend à la fois un fragment Fab spécifiquement immuno-réactif vis-à-vis de l'antigène 18 des souches méthicilline-résistantes, et un fragment Fc susceptible de se fixer sur la protéine A desdites bactéries.

9. Procédé selon la revendication 8, caractérisé en ce que ledit anticorps est de type polyclonal, ou tout autre anticorps présentant une réaction immunologique croisée avec ledit anticorps.

10. Procédé selon la revendication 8, caractérisé en ce que ledit anticorps est de type monoclonal, ou tout autre anticorps présentant une réaction immunologique croisée avec ledit anticorps.

11. Procédé selon la revendication 10, caractérisé en ce qu'on injecte à une espèce animale une culture de cellules de la lignée cellulaire hybridome telle que déposées le 26 juillet 1991, sous le N° 91072636 auprès de l'ECACC, ou toute autre lignée cellulaire dérivée, ou toute progéniture de cet hybridome, pour autant que ces lignées et progéniture soient capables de produire ledit anticorps, et on extrait l'anticorps du liquide ascite récolté sur ladite espèce.

12. Procédé selon la revendication 10, caractérisé en ce qu'on injecte à une espèce animale une culture des cellules de la lignée cellulaire hybridome résultant de la fusion d'une cellule provenant de lignée cellulaire Sp2/OAg14 (ATCC CRL 1581), avec une cellule de rate de souris provenant d'une souris immunisée avec le sérotype 18 de la souche de *Staphyloccocus aureus,* susceptible de produire ledit anticorps.

13. Procédé selon la revendication 11 ou 12, du type agglutination.

## Patentansprüche (Patentansprüche für folgende(n) Vertragsstaat(en): AT, BE, CH, DE, FR, GB, IT, LI)

1. Reagenz zur Identifizierung von Bakterien der Art *Staphylococcus aureus,* insbesondere der gegen Methicillin resistenten Stämme, welches eine reaktive Zusammensetzung des Immunoglobulin-Types aufweist, die einen Fc-Abschnitt aufweist, der sich auf dem Protein A dieser Bakterien festsetzen kann und ggf. eine andere Zusammensetzung auf Basis von Fibrinogen oder von dessen Derivaten aufweist, die mit dem Affinitätsfaktor für das Fibrinogen dieser Bakterien reagieren kann, dadurch gekennzeichnet, daß die reaktive Zusammensetzung, neben dem Fc-Abschnitt einen gegenüber dem Antigen 18 der methicillinresistenten Stämme spezifisch immunoreaktiven Fab-Abschnitt aufweist.

2. Reagenz nach Anspruch 1, dadurch gekennzeichnet, daß die reaktive Zusammensetzung einen Antikörper, ggf. auf einem Träger oder einem Substrat fixiert, enthält, der die Abschnitte Fab und Fc aufweist.

3. Reagenz nach Anspruch 2, dadurch gekennzeichnet, daß der Antikörper an Partikel eines Polymeren, insbesondere eines Elastomeren, beispielsweise einem Latex, oder an magnetische Partikel fixiert ist.

4. Reagenz nach Anspruch 2, dadurch gekennzeichnet, daß der Antikörper an menschliche oder tierische Erythrozyten fixiert ist.

5. Reagenz nach Anspruch 1, dadurch gekennzeichnet, daß es die andere Zusammensetzung auf Basis von Fibrinogen oder von dessen Derivaten enthält, die wiederum auf einem Träger oder einem Substrat fixiert ist.

6. Reagenz nach Anspruch 1, dadurch gekennzeichnet, daß die reaktive Zusammensetzung und die andere Zusammensetzung auf Basis von Fibrinogen auf ein und demselben Träger oder Substrat fixiert sind, und daß die reaktive Zusammensetzung ein Antikörper ist.

7. Reagenz nach Anspruch 1, bei dem die reaktive Zusammensetzung ein Antikörper ist und die andere Zusammensetzung auf Basis von Fibrinogen vorhanden ist, wobei die beiden Zusammensetzungen jeweils auf unterschiedlichen Trägern fixiert sind, insbesondere auf Latexpartikeln bzw. auf Erythrozyten.

8. Antikörper vom Immunoglobulin-Typ, der gegenüber Bakterien der Art *Staphylococcus aureus* spezifisch ist, insbesondere gegenüber gegen Methicillin resistenten Stämmen, dadurch gekennzeichnet, daß er vom Immunoglobulin-Typ, insbesondere der Klasse IgG, ist und zugleich einen Fab-Abschnitt, der gegenüber dem Antigen 18 der methicillinresistenten Stämme spezifisch immunoreaktiv ist und einen Fc-Abschnitt enthält, der sich auf dem Protein A dieser Bakterien festsetzen kann.

9. Antikörper nach Anspruch 8, der polyklonal ist oder ein jeglicher anderer Antikörper ist, der mit diesem Antikörper eine gekreuzte immunologische Reaktion aufweist, dadurch gekennzeichnet, daß er dadurch erhalten wurde, daß ein Tier mit dem Antigen 18 immunisiert wurde und der Antikörper vom so erhaltenen Plasma extrahiert worden ist.

10. Antikörper nach Anspruch 8, der monoklonal ist oder ein jeglicher anderer Antikörper ist, der mit diesem Antikörper eine gekreuzte immunologische Reaktion aufweist, dadurch gekennzeichnet, daß er aus der Hybridom-Zellinie erhalten wurde, wie sie am 26. Juli 1991 unter der Nummer 91072636 bei der ECACC hinterlegt worden ist.

11. Hybridom-Zellinie, wie sie am 26. Juli 1991 unter der Nummer 91072637 bei der ECACC hinterlegt worden ist oder jegliche andere derivate Zellinie oder jegliche Abkömmlinge dieses Hybridoms, vorausgesetzt, daß diese Linien oder Abkömmlinge in der Lage sind, einen Antikörper nach Anspruch 10 zu erzeugen.

12. Hybridom-Zellinie, resultierend aus der Fusion einer Zelle, die aus der Zellinie Sp2/OAg14 (ATCC CRL 1581) herstammt, mit einer Mäusemilzzelle, die von einer Maus stammt, die mit dem Serotyp 18 des Stammes des *Staphylococcus aureus* immunisiert ist, die einen Antikörper nach Anspruch 10 erzeugen kann.

13. Verfahren zum Herstellen eines Antikörpers nach Anspruch 10, dadurch gekennzeichnet, daß man einer Tierspezies, soweit diese vom Hybridom abstammt, eine Zellkultur, wie sie am 26. Juli 1991 unter der Nummer 91072637 bei der ECACC hinterlegt worden ist, injiziert und daß der Antikörper aus der von der genannten Spezies gewonnenen Ascites-Flüssigkeit extrahiert wird.

14. Verfahren nach Anspruch 13, vom Agglutinations-Typ.

15. Verfahren zur Identifizierung von Bakterien der Art *Staphylococcus aureus,* einschließlich der gegen Methicillin resistenten Stämme, bei dem ein Komplex zwischen einer reaktiven Zusammensetzung und einem Antigen dieser Bakterien gebildet wird, dadurch gekennzeichnet, daß die reaktive Zusammensetzung vom Immunoglobulin-Typ ist, und zugleich einen gegenüber dem Antigen 18 der methicillinresistenten Stämme spezifisch immunoreaktiven Fab-Abschnitt und einen Fc-Abschnitt aufweist, der aufweist, der sich auf dem Protein A dieser Bakterien footootzen kann.

## Patentansprüche (Patentansprüche für folgende(n) Vertragsstaat(en): ES)

1. Verfahren zur Identifizierung von Bakterien der Art *Staphylococcus aureus,* einschließlich der gegen Methicillin resistenten Stämme, bei dem ein Komplex zwischen einer reaktiven Zusammensetzung und einem Antigen dieser Bakterien gebildet wird, und ggf. ein Komplex mit einer anderen Zusammensetzung auf Basis von Fibrinogen oder von dessen Derivaten gebildet wird, welche mit dem Affinitätsfaktor für das Fibrinogen dieser Bakterien reagieren kann, dadurch gekennzeichnet, daß die reaktive Zusammensetzung vom Immunoglobulin-Typ ist, und zugleich einen gegenüber dem Antigen 18 der methicillinresistenten Stämme spezifisch immunoreaktiven Fab-Abschnitt und einen Fc-Abschnitt aufweist, der sich auf dem Protein A dieser Bakterien festsetzen kann.

2. Verfahren nach Anspruch 1, dadurch gekennzeichnet, daß die reaktive Zusammensetzung einen Antikörper, ggf. auf einem Träger oder einem Substrat fixiert, aufweist, der die Abschnitte Fab und Fc aufweist.

3. Verfahren nach Anspruch 2, dadurch gekennzeichnet, daß der Antikörper an Partikel eines Polymeren, insbesondere eines Elastomeren, beispielsweise einem Latex, oder an magnetische Partikel fixiert ist.

4. Verfahren nach Anspruch 2, dadurch gekennzeichnet, daß der Antikörper an menschliche oder tierische Erythrozyten fixiert ist.

5. Verfahren nach Anspruch 1, dadurch gekennzeichnet, daß die andere Zusammensetzung auf Basis von Fibrinogen oder von dessen Derivaten selbst auf einem Träger oder einem Substrat fixiert ist.

6. Verfahren nach Anspruch 1, dadurch gekennzeichnet, daß die reaktive Zusammensetzung und die andere Zusammensetzung auf Basis von Fibrinogen auf ein und demselben Träger oder Substrat fixiert sind, und daß die reaktive Zusammensetzung ein Antikörper ist.

7. Verfahren nach Anspruch 1, bei dem die reaktive Zusammensetzung ein Antikörper ist und die andere Zusammensetzung auf Basis von Fibrinogen vorhanden ist, wobei die beiden Zusammensetzungen jeweils auf unterschiedlichen Trägern fixiert sind, insbesondere auf Latexpartikeln bzw. auf Erythrozyten.

8. Verfahren zur Herstellung eines Antikörpers vom Immunoglobulin-Typ, der gegenüber Bakterien der Art *Staphylococcus aureus,* insbesondere der gegen Methicillin resistenten Stämme, spezifisch ist, dadurch gekennzeichnet, daß ein Tier mit dem Antigen 18 immunisiert wird, daß man aus dem so erhaltenen Plasma die Antikörper extrahiert, und daß der Antikörper vom Immunoglobulin-Typ, insbesondere der Klasse IgG, ist und zugleich einen Fab-Abschnitt, der gegenüber dem Antigen 18 der methicillinresistenten Stämme spezifisch immunoreaktiv ist und einen Fc-Abschnitt enthält, der sich auf dem Protein A dieser Bakterien festsetzen kann.

9. Verfahren nach Anspruch 8, dadurch gekennzeichnet, daß der Antikörper polyklonal ist oder ein jeglicher anderer Antikörper ist, der mit diesem Antikörper eine gekreuzte immunologische Reaktion aufweist.

10. Verfahren nach Anspruch 8, dadurch gekennzeichnet, daß der Antikörper monoklonal ist oder ein jeglicher anderer Antikörper ist, der mit diesem Antikörper eine gekreuzte immunologische Reaktion aufweist.

11. Verfahren nach Anspruch 10, dadurch gekennzeichnet, daß man einer Tierspezies eine Zellkultur der Hybridom-Zellinie, wie sie am 26. Juli 1991 unter der Nummer 91072636 bei der ECACC hinterlegt wurde, oder jegliche andere derivate Zellinie oder jegliche Abkömmlinge dieses Hybridoms injiziert, vorausgesetzt, daß diese Linien oder Abkömmlinge in der Lage sind, den Antikörper zu erzeugen, und daß der Antikörper aus der von der genannten Spezies gewonnenen Ascites-Flüssigkeit extrahiert wird.

12. Verfahren nach Anspruch 10, dadurch gekennzeichnet, daß man einer Tierspezies eine Zellkultur der Hybridom-Zellinie injiziert, die aus der Fusion einer Zelle, die aus der Zellinie Sp2/OAg14 (ATCC CRL 1581) herstammt, mit einer Mäusemilzzelle resultiert, die von einer Maus stammt, die mit dem Serotyp des Stammes des *Staphylococcus aureus* immunisiert ist, der diese Antikörper herstellen kann.

13. Verfahren nach Anspruch 11 oder 12 vom Agglutinations-Typ.

## Claims (Claims for the following Contracting State(s): AT, BE, CH, DE, FR, GB, IT, LI)

1. Reagent for the identification of bacteria of the species *Staphyloccocus (sic) aureus,* especially of the strains resistant to methicillin, of the type comprising a reactive compound, of the immunoglobulin type, comprising an Fc fragment capable of attaching to protein A of the said bacteria, and optionally another compound based on fibrinogen or a derivative, capable of reacting with the fibrinogen affinity factor of the said bacteria, **characterized in that** the reactive compound comprises, in addition to the Fc fragment, a Fab fragment specifically immunoreactive towards antigen 18 of the methicillin-resistant strains.

2. Reagent according to Claim 1, characterized in that the reactive compound comprises an antibody containing the said Fab and Fc fragments, which is optionally attached to a support or substrate.

3. Reagent according to Claim 2, characterized in that the antibody is attached to particles of a polymer, especially an elastomer, for example a latex, or to magnetic particles.

4. Reagent according to Claim 2, characterized in that the antibody is attached to human or animal red blood cells.

5. Reagent according to Claim 1, characterized in that it comprises the said other compound based on fibrinogen or a derivative, itself attached to a support or substrate.

6. Reagent according to Claim 1, containing the reactive compound and the other compound based on fibrinogen, attached to the same support or substrate, the reactive compound being an antibody.

7. Reagent according to Claim 1, in which the reactive compound is an antibody, and the other compound based on fibrinogen is present, these two compounds being respectively attached to different supports, especially latex particles and red blood cells respectively.

8. Antibody of the immunoglobulin type, specific for bacteria of the species *Staphylococcus aureus,* and especially the strains resistant to methicillin, characterized in that it is of the immunoglobulin type, especially of the IgG class, and comprises both a Fab fragment specifically immunoreactive towards antigen 18 of the methicillin-resistant strains, and an Fc fragment capable of attaching to protein A of the said bacteria.

9. Antibody according to Claim 8, of the polyclonal type, or any other antibody exhibiting immunological cross-reactivity with the said antibody, characterized in that it is obtained by immunizing an animal with antigen 18, and by extracting the antibody from the plasma thus obtained.

10. Antibody according to Claim 8, of the monoclonal type, or any other antibody exhibiting immunological cross-reactivity with the said antibody, characterized in that it is obtained from the hybridoma cell line deposited on 26 July 1991 under No. 91072636 at the ECACC.

11. Hybridoma cell line, as deposited on 26 July 1991 under No. 91072636 at the ECACC, or any other derived cell line, or any progeny of this hybridoma, as long as these lines and progeny are capable of producing an antibody according to Claim 10.

12. Hybridoma cell line resulting from the fusion of a cell obtained from the cell line Sp2/OAg14 (ATCC CRL 1581), with a mouse spleen cell obtained from a mouse immunized with serotype 18 of the *Staphyloccocus (sic) aureus* strain, capable of producing an antibody according to Claim 10.

13. Process for the production of an antibody according to Claim 10, characterized in that an animal species from which the hybridoma is derived is injected with a culture of the cells as deposited on 26 July 1991, under No. 91072636 at the ECACC, and the antibody is extracted from the ascitic fluid harvested from the said species.

14. Process according to Claim 13, of the agglutination type.

15. Process for the identification of bacteria of the species *Staphylococcus aureus,* including the strains resistant to methicillin, according to which a complex is formed between a reactive compound and an antigen of the said bacteria, characterized in that the reactive compound is of the immunoglobulin type, and comprises both a Fab fragment specifically immunoreactive towards antigen 18 of the methicillin-resistant strains, and an Fc fragment capable of attaching to protein A of the said bacteria.

## Claims (Claims for the following Contracting State(s): ES)

1. Process for the identification of bacteria of the species *Staphylococcus aureus,* including the strains resistant to methicillin, according to which a complex is formed between a reactive compound and an antigen of the said bacteria, and a complex is optionally formed between another compound based on fibrinogen or a derivative, capable of reacting with the fibrinogen affinity factor of the said bacteria, characterized in that the reactive compound is of the immunoglobulin type, and comprises both a Fab fragment specifically immunoreactive towards antigen 18 of the methicillin-resistant strains, and an Fc fragment capable of attaching to protein A of the said bacteria.

2. Process according to Claim 1, characterized in that the reactive compound comprises an antibody containing the said Fab and Fc fragments, which is optionally attached to a support or substrate.

3. Process according to Claim 2, characterized in that the antibody is attached to particles of a polymer, especially an elastomer, for example a latex, or to magnetic particles.

4. Process according to Claim 2, characterized in that the antibody is attached to human or animal red blood cells.

5. Process according to Claim 1, characterized in that the said other compound based on fibrinogen or a derivative is itself attached to a support or substrate.

6. Process according to Claim 1, characterized in that the reactive compound and the other compound based on fibrinogen are attached to the same support or substrate, and in that the reactive compound is an antibody.

7. Process according to Claim 1, according to which the reactive compound is an antibody, and the other compound based on fibrinogen is present, these two compounds being respectively attached to different supports, especially latex particles and red blood cells respectively.

8. Process for the production of an antibody of the immunoglobulin type, specific for bacteria of the species *Staphylococcus aureus,* and especially strains resistant to methicillin, characterized in that an animal is immunized with antigen 18 and the antibody is extracted from the plasma thus obtained, and in that the said antibody is of the immunoglobulin type, especially of the IgG class, and comprises both a Fab fragment specifically immunoreactive towards antigen 18 of the methicillin-resistant strains, and an Fc fragment capable of attaching to protein A of the said bacteria.

9. Process according to Claim 8, characterized in that the said antibody is of the polyclonal type, or any other antibody exhibiting immunological cross-reactivity with the said antibody.

10. Process according to Claim 8, characterized in that the said antibody is of the monoclonal type, or any other antibody exhibiting immunological cross-reactivity with the said antibody.

11. Process according to Claim 10, characterized in that an animal species is injected with a culture of cells of the hybridoma cell line as deposited on 26 July 1991, under No. 91072636 at the ECACC, or any other derived cell line, or any progeny of this hybridoma, as long as these lines and progeny are capable of producing the said antibody, and the antibody is extracted from the ascitic fluid harvested from the said species.

12. Process according to Claim 10, characterized in that an animal species is injected with a culture of cells of the hybridoma cell line resulting from the fusion of a cell obtained from the cell line Sp2/OAg14 (ATCC CRL 1581), with a mouse spleen cell obtained from a mouse immunized with serotype 18 of the *Staphylococcus aureus* strain, capable of producing the said antibody.

13. Process according to Claim 11 or 12, of the agglutination type.
